# EUROPEAN PATENT APPLICATION

(11) **EP 1 255 114 A1**
(43) Date of publication of application: **06.11.2002**
(21) Application number: 01904327.2
(22) Date of filing: 07.02.2001
(51) Int. Cl.: G01N 33/92, G01N 33/493

(54) **METHOD OF MEASURING LIPID COMPONENTS AND METHOD OF EXAMINING RENAL FAILURE**

(30) Priority: 08.02.2000 JP 2000030980; 13.07.2000 JP 2000212431
(71) Applicant: INTERNATIONAL REAGENTS CORPORATION, Kobe-shi Hyogo 651-0083 (JP)
(72) Inventor: HOTTA, Osamu, Sendai-shi, Miyagi 983-0823 (JP); SHIRAHASE, Yasushi International Reagents Corp., Kobe-shi, Hyogo 651-2241 (JP); HIURA, Hisahide R&DC, International Reagents Corp., Kobe shi, Hyogo 651-2241 (JP)
(74) Representative: Forstmeyer, Dietmar, Dr. rer. nat., Dipl.-Chem.
(86) International application number: JP0100847
(87) International publication number: WO01059462

(57) **Abstract**

The problems of the present invention are to provide a simple and convenient means for the examination of renal failure and to provide a method for the measurement of a lipid component in urine utilizing the said examining means.

Means for solving the problems according to the present invention is a method for the measurement of a lipid component contained in urine and it provides a method for measurement of a lipid component in urine which is characterized in using a surface-active agent in a sufficient amount for solubilizing the insoluble fat in urine and in using an enzyme acting the lipid component. It also provides a simple and convenient means for the examination of renal failure by the measurement of a lipid component contained in urine, by the measurement of lipoproteins in urine and/or apoproteins in urine in addition to the above measurement and by a combination of measurement of surface antigen of leukocyte contained in urine therewith.

## Description

### Background of the Invention

### 1. Field of the Invention

The present invention relates to a method for the measurement of lipid component in urine and to a reagent for the measurement. The present invention further relates to a examination means for renal failure utilizing the said method for the measurement.

### 2. Description of the Related Art

Components contained in urinary sediment are diversified such as components derived from kidney, components mingled from urinary tract and crystalline components separated out in urine. Checking the type and the amount of the sediment is very important in judging the diseases of kidney and urinary tract and in knowing their degree. They are usually carried out by an observation under a microscope by a dyeing method. There are also shown that pathological diagnosis of IgA nephropathy is possible by checking the surface antigen of macrophage in urine *(Rinsho Kensa,* volume 42, 588∼590, 1998) and that examination of renal failure is possible by judging the fat particle and oviform fat body (hereinafter, sometimes referred to as just "fat body") in urine by means of dyeing of fat in the urinary sediment(*Kensa to Gijutsu,* volume 26, 441-446, 1998).

Further, in macrophage in a big size appeared in urine, surface antigen CD14 of leukocyte is negative while 25F9 is positive and that is greatly different from the usual macrophage where CD14 is positive while 25F9 is negative. In addition, it is shown that fat particle and oviform fat body belong to microphage in a big size and it is mentioned that measurement of them is useful as the examination for renal failure *(Rinsho Byori,* volume 47, Issue for General Meeting, 73, 1999).

However, all of those measurements have been carried out by observation under a microscope by dyeing, observation under a microscope by fluorescence labeled immunodyeing using monoclonal antibody or flow cytometry. In such a method, each sample is examined under a microscope or by a flow cytometry and, therefore, the operation is complicated and measurement of many samples is difficult.

### Summary of the Invention

An object of the present invention is to provide a simple and convenient means for the examination of renal failure. The present inventors have carried out an intensive investigation for solving the above-mentioned problems and found that the amount of the lipid component in urine significantly suggests the relationship with the renal failure.

Since the lipid component in urine is in small amount as compared with the amount of lipid component. contained in serum, a method for the measurement of lipid component having a high sensitivity is demanded. In view of the above, the present inventors have carried out further investigation and, as a result, they have paid their attention to fat granules contained in the fat particle and oviform fat body in urine which are observed in connection with renal failure and have found that measurement of the lipid component contained therein can be made possible by solubilizing them using a surface-active agent whereupon they have accomplished a method for measuring the lipid component contained in urine in accordance with the present invention. Incidentally, the meaning of "solubilizing" in the present invention will be mentioned later.

Thus, the present invention comprises the followings.
1. A means for the examination of renal failure which is characterized in measuring a lipid component contained in urine.
2. The means for the examination of renal failure according to the above 1, wherein the lipid component contained in the urine is measured by the use of enzyme acting the lipid component.
3. The means for the examination of renal failure according to the above 2, wherein the enzyme acting the lipid component is one or more enzyme(s) selected from enzymes which catalyze decomposition reaction, dehydration reaction or oxidation reaction.
4. The means for the examination of renal failure according to any of the above 1∼3, wherein the said lipid component is contained in at least one or more of fat drop, fat body and fat particle in urine.
5. The means for the examination of renal failure according to any of the above 1∼4, wherein the said lipid component is one or more lipid component(s) selected from neutral fat and derivatives thereof, phospholipidand derivatives thereof, lipid peroxide, sterols, fatty acid, fatty acid salt, fatty acid ester, aliphatic alcohol, aliphatic aldehyde, glycolipid, sphingolipid, prostaglandin and carotenoid.
6. A method for the measurement of a lipid component contained in urine, characterized in that, a means where insoluble fat in urine is solubilized or dispersed to homogenize is used.
7. The method for the measurement according to the above 6, wherein the means where insoluble fat in urine is solubilized or dispersed to homogenize is to use a surface-active agent.
8. The method for the measurement according to the above 6 or 7, wherein the lipid component is contained in at least one or more of fat drop, fat body or fat particle.
9. The method for the measurement according to any of the above 6∼8, wherein the said lipid component is one or more lipid component (s) selected from neutral fat and derivatives thereof, phospholipid and derivatives thereof, lipid peroxide, sterols, fatty acid, fatty acid salt, fatty acid ester, aliphatic alcohol, aliphatic aldehyde, glycolipid, sphingolipid, prostaglandin and carotenoid.
10. The method for the measurement according to any of the above 6∼9, wherein the said lipid component is any of the sterols and phospholipid.
11. The method for the measurement according to any of the above 6∼10, wherein the said surface-active agent has an action of solubilizing at least one or more of any of fat drop, fat body and fat particle and is able to solubilize, disperse, liberate and homogenize a lipid component contained in any of the said fat drop, fat body and fat particle.
12. The method for the measurement according to any of the above 6∼11, wherein the said surface-active agent is nonionic surface-active agent, cationic surface-active agent, anionic surface-active agent, amphoteric surface-active agent or glycoside.
13. The method for the measurement according to any of the above 6∼12, wherein two or more of the said surface-active agents are jointly used.
14 . The means for the examination of renal failure according to the above 1, wherein the method mentioned in any of the above 6∼13 is utilized.
15. The means for the examination of renal failure according to the above 1, wherein the method mentioned in any of the above 6∼13 is further utilized in addition to the means for the examination mentioned in the above 2 or 3.
16. The means for the examination of the renal failure according to any of the above 1∼3, 14 and 15, wherein the means is carried out in combination with at least one or more measurement(s) of lipoprotein in urine or apoprotein in urine.
17 . The means for the examination of renal failure according to any of the above 1∼3 and 14∼16, wherein the means is carried out in combination with measurement or detection of surface antigen of leukocyte contained in urine.
18. A reagent for the measurement of lipid component in urine where the reagents necessary for the method for the measurement mentioned in any of the above 6∼13 are made in a kit or are composed of each single substance.

The present invention is a means for the examination of renal failure and is characterized in measuring a lipid component contained in urine. Here, the term "means" stands for a method and/or a medium for achieving the object.

With regard to a method for the measurement of a lipid component in small amount in urine, the present inventors have further found that the measurement of the lipid component in urine is also possible by the use of a surface-active agent which is sufficient for solubilizing or dispersing to homogenize the insoluble fat in urine.

The lipid component in urine measured by the method of the present invention for the measurement of lipid component contained in urine is a lipid component mainly contained in fat drop, fat body and/or fat sphere and may be one or more lipid component (s) selected from neutral fat and derivatives thereof, phospholipid and derivatives thereof, lipid peroxide, sterols, fatty acid, fatty acid salt, fatty acid ester, aliphatic alcohol, aliphatic aldehyde, glycolipid, sphingolipid, prostaglandin and carotenoid.

The enzyme used in the method of the present invention for the measurement of a lipid component contained in urine may be one or more enzyme(s) selected from enzymes for catalyzing decomposition reaction, dehydration reaction or oxidization reaction.

The surface-active agent used in the method of the present invention for the measurement of a lipid component in urine may be anything which has an action of solubilizing or dispersing fat drop, fat body and fat particle to homogenize and is able to solubilize, disperse, liberate and homogenize the lipid component contained in the said fat drop, fat body and fat particle. With regard to the surface-active agent, two or more kinds thereof may be used jointly or it may be nonionic surface-active agent, cationic surface-active agent, anionic surface-active agent, amphoteric surface-active agent or glycoside.

The means of the present invention for the examination of renal failure is characterized in utilizing the above-mentioned method of the present invention for the measurement of a lipid component contained in urine and the lipid component to be measured may be that which is contained in fat drop, fat body and/or fat particle in urine or may be one or more lipid component(s) selected from neutral fat and derivatives thereof, phospholipid and derivatives thereof, lipid peroxide, sterols, fatty acid, fatty acid salt, fatty acid ester, aliphatic alcohol, aliphatic aldehyde, glycolipid, sphingolipid, prostaglandin and carotenoid.

The means of the present invention for the exdamination of renal failure may be carried out by combining the above-mentioned method of the present invention for the measurement of lipid component containing in urine with measurement of lipoprotein and/or apoprotein in urine and measurement of detection of surface antigen of leukocyte contained in urine.

The present invention further provides a reagent for the measurement of lipid component in urine where the reagents necessary for the above-mentioned method for the measurement mentioned are made in a kit or are composed of each single substance.

### Brief Description of the Drawings

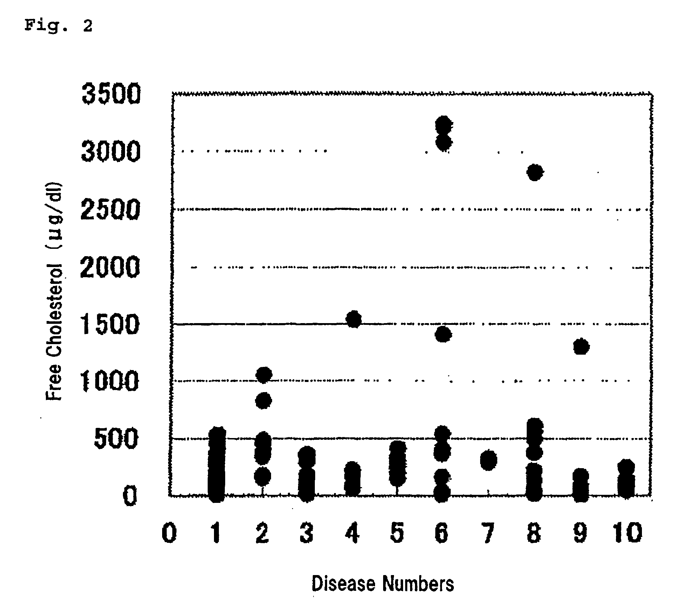

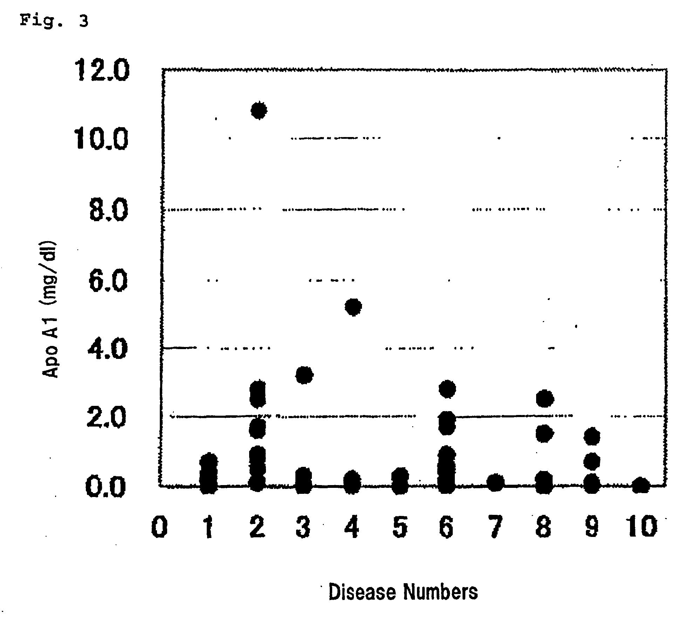

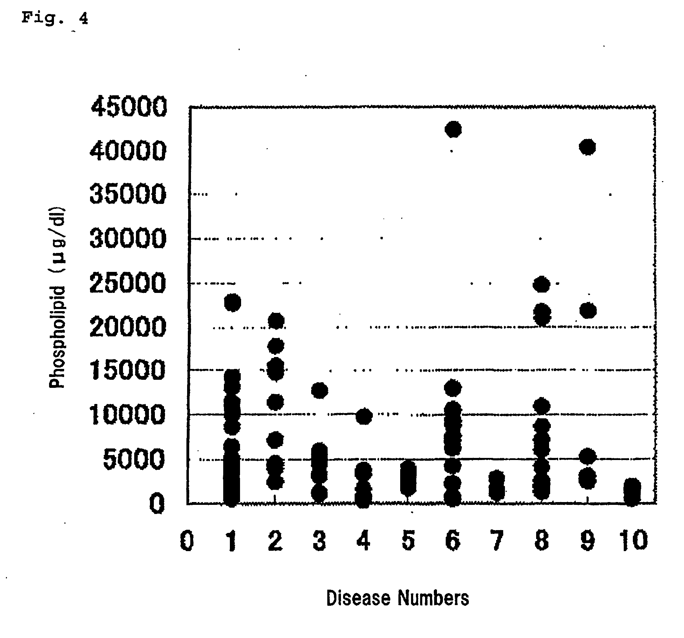

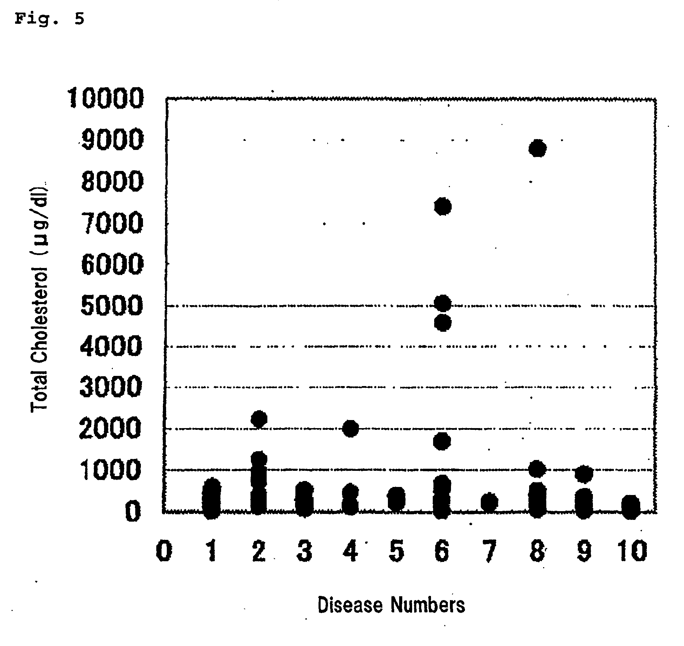

Phospholipid, free cholesterol, cholesterol and apo A1 were measured for 122 urine samples and the relation thereof with the disease was investigated. Fig. 1 shows the relation of lipid-related marker (Oilred-positive particles) in urine with the disease; Fig. 2 shows the relation of lipid-related marker (free cholesterol) in urine with the disease; Fig. 3 shows the relation of lipid-related marker (apo A1) in urine with the disease; Fig. 4 shows the relation of lipid-related marker (phospholipid) in urine with the disease; and Fig. 5 shows the relation of lipid-related marker (total cholesterol) in urine with the disease. In the drawings, 1 is IgA nephropathic disease; 2 is diabetic nephropathic disease; 3 is nephrosclerotic disease; 4 is membranous nephropathic disease; 5 is membranous proliferative glomerulonephritic disease; 6 is membranous proliferative glomerulonephritic disease; 7 is minimal change nephrotic syndrome disease; 8 is focal glomerular sclerotic disease; 9 is chronic renal failure disease; and 10 is normal.

### Detailed Description of Preferred Embodiments

The means according to the present invention for solving the problems is to measure a lipid component contained in urine.

With regard to a novel method for the measurement, there is listed a method for the measurement of a lipid component in urine which is characterized in containing the use of surface-active agent being sufficient for solubilizing or dispersing the insoluble fat in urine to homogenize and the use of enzyme acting the lipid component.

Here, the term "solubilizing" or "solubilizing or dispersing to homogenize" means that the lipid component which is insoluble in urine is made to exist in the urine in a stable and homogeneous state. Thus, in the urine where the lipid component is solubilized or it is solubilized or dispersed to homogenize, the lipid component exists in nearly the same concentration when any part of the urine is collected and subjected to the measurement of lipid. Another meaning for the expression reading the lipid component is solubilized or it is solubilized or dispersed to homogenize is that the lipid is made into a state which is able to be subjected to a quantitative determination by an enzymatic reaction as a result of the action of a surface-active agent. The said expression may have one of the meanings or may have both meanings. In the measurement of the lipid component, a known method which will be mentioned later is used.

With regard to the lipid component in urine, the main object to be measured is that which is contained in fat drop, fat body and/or fat particle. In the serum, lipid exists in a solubilized state while, in the urine, it is often in a state of particles in a degree of being able to be observed under a microscope. In accordance with the present invention, the thing which is in a state of an insoluble fat lump as such is able to be quantitatively determined for the amount of lipid component by an enzymatic reaction.

### (Lipid Component to be Measured)

Examples of the lipid component in urine which is to be measured by the present invention are one or more lipid component (s) selected from neutral fat and derivatives thereof, phospholipid and derivatives thereof, lipid peroxide, sterols, fatty acid, fatty acid salt, fatty acid ester, aliphatic alcohol, aliphatic aldehyde, glycolipid, sphingolipid, prostaglandin and carotenoid and the concentration or the content thereof is measured by treating with an enzyme which acts the said lipid component. Among the lipid components, particularly important objects are sterols and/or phospholipid.

### (Treatment of Samples to be Measured)

The urine sample may be either the urine *per se* or a urinary sediment fraction or a supernatant fraction thereof obtained by centrifugal separation of urine. In the method of the present invention for the measurement of lipid component, it is possible that the urine sample is directly measured without any treatment and, moreover, it is also possible that urine is centrifuged to fractionate the urinary sediment and the lipid component in the said fraction is measured.

### (Addition of Surface-Active Agent)

In the present invention, the lipid component contained in the urine sample is measured by treating with an enzyme acting the lipid component in the presence of a surface-active agent. With regard to the surface-active agent, although there is no particular limitation so far as it is a substance having an action of solubilizing or dispersing the insoluble fat in the urine to homogenize, a substance which has a solubilizing action for fat drop, fat body and fat particle and is able to solubilize, disperse and liberate the lipid component contained therein. The lipid component may also be solubilized, dispersed and liberated to homogenize by means of an ultrasonic wave treatment either solely or in combination with the treatment using a surface-active agent.

In the specific examples of the present invention, Triton X-100 is exemplified as a nonionic surface-active agent although that is not limitative. On the basis of the result disclosed in the present invention, the persons skilled in the art are able to compare the effect by means of simple experimental repetition and to find a surface-active agent which is able to solubilize or disperse the insoluble fat for making it homogeneous and is effective for the measurement of lipid component contained in the urine. Besides the nonionic surface-active agent, it is of course possible to appropriately use cationic surface-active agent, anionic surface-active agent, amphoteric surface-active agent or glycoside. It is further possible to use two or more surface-active agents jointly for solubilize or disperse more surely to achieve the homogenizing effect.

With regard to the adding amount of the surface-active agent, it is common to use 0.1∼5 w/v% although that may be selected by an appropriate increase or decrease taking the degree of homogenization due to solubilization or dispersing of the lipid component into consideration. Since temperature, time, etc. which are the treating conditions depend upon the reaction condition of the lipid component with the enzyme, it is preferred to select the optimum condition for the lipid component which is the object to be measured and for the enzyme and that may be decided by a simple experimental repetition. Preferably, it is also possible that, as a pretreatment, surface-active agent is added previously so that the lipid component is solubilized or dispersed to carry out the homogenizing treatment. In that case, a treatment for from about several seconds to about several minutes is sufficient at the temperature of 10∼40°C. Needless to say, the said treatment with the surface-active agent may be carried out together with the enzymatic reaction treatment. Although the treatment may be conducted by means of an appropriate stepwise treatment, it is preferred to carry out under an automated condition or to conduct a unified treatment.

### (Quantitative Determination of Lipid Component Using Enzyme)

As mentioned above, measurement of the lipid component in urine which is solubilized or dispersed by addition of a surface-active agent or the like to homogenize is carried out by means of a quantitative determination method for an enzyme and there is no particular limitation for such a method so far as it is a known method. Thus, there may be exemplified HPLC method, gas chromatographic method, electrophoretic method, chemical method and method using enzyme. Among them, the method using enzyme is advantageously used in view of its operation and sensitivity. In a method for the measurement of the lipid component according to the present invention, it is possible to measure the lipid component utilizing one or more enzyme(s) selected from enzymes which catalyze decomposition reaction, dehydration reaction or oxidation reaction as will be shown in the following specific examples. As compared with the lipid component in serum, that in urine is present only a little and its measurement with a high sensitivity is possible by the use of enzyme.

To be more specific, in an example where neutral fat is measured, the neutral fat is decomposed with lipase into glycerol and fatty acid and the resulting glycerol is measured by an enzymatic reaction. Measurement of glycerol by the enzymatic reaction may be carried out by a method where glycerol kinase and glycerol-3-phosphate dehydrogenase are used and the rate of change from coenzyme NAD to NADH is measured; a method where glycerol kinase and glycerol phosphoxidase are used and the resulting hydrogen peroxide is measured; a high-sensitivity method where glycerol kinase, glycerol-3-phosphate dehydrogenase and glycerol phosphoxidase are used and glycerol is measured by a cycling reaction; a method where glycerol dehydrogenase is used for the measurement; etc.

In the measurement of phospholipid, there may be used a method where glycerol 3-phosphate is produced. by the action of phospholipase and glycerophosphorylcholine diesterase and the said glycerol 3-phosphate is measured by the same manner as in the case of neutral fat and other known methods where phospholipid is enzymatically measured.

In the measurement of cholesterol which is a representative sterol in living body, there have been known a method where cholesterol oxidase is used to produce hydrogen peroxide and the resulting hydrogen peroxide is measured by peroxidase; a method where cholesterol dehydrogenase is treated and the changing rate or the changed amount of a coenzyme NAD to NADH is measured; a method where cholesterol dehydrogenase is used and an equilibrium amplification reaction using two kinds of coenzymes NADH and thio-NAD is carried out whereby cholesterol is measured in a high sensitivity; etc. (Japanese Patent Laid-Open No. 08/70,894). There has been also reported a method where a cycling reaction is carried out using cholesterol as a substrate in the presence of a reduced type substrate of the second dehydrogenase which regenerates a coenzyme of an oxidizing type being converted by cholesterol oxidase, etc. to a coenzyme of a reducing type whereupon the measurement is carried out (Japanese Patent Laid-Open No. 11/18,797).

In the case of fatty acid ester of cholesterol, it is possible that a decomposition enzyme such as cholesterol esterase for converting to free cholesterol is used and the measurement is carried out by the above-mentioned method.

In the case of fatty acid, there is used a method where acyl coenzyme A synthetase and acyl coenzyme A oxidase are used and the resulting hydrogen peroxide is measured.

In the case of prostaglandin, it is possible to conduct the measurement where a prostaglandin dehydrogenase is used.

In the case of glycolipid, it is possible to conduct the measurement where a glycolipid-decomposing lipase and other lipase are used and the resulting glycerol is measured.

In the case of sphingolipid, it is possible to conduct the measurement where a sphingolipid-decomposing lipase is used.

In the case of lipoprotein, it is possible to conduct the measurement adopting the above-mentioned method for the lipid fraction contained in the lipoprotein.

On the other hand, with regard to the measurement of apoprotein, it is possible to conduct the measurement by an antigen-antibody reaction. In the case of apo B protein for example, there may be adopted known measuring methods such as immunoturbidimetry, particle aggregation immunoassay and enzymatic immunoassay. Examples of other apo protein are apo A-I, apo A-II, apo C, apo E and apo LP(a). Further, measurement of a lipid component corresponding to the type of the lipoprotein is also possible by adopting the known methods.

Methods for the measurement of a lipid component are not limited to the above-exemplified methods but appropriate change and addition are possible according to the known measuring methods for a lipid component.

In the present invention, there is also provided a reagent for the measurement of a lipid component in urine where the reagents necessary for the method for the measurement according to the present invention mentioned hereinabove are made in a kit or are composed of each single substance. The reagent of the present invention for the measurement of a lipid component in urine contains a surface-active agent for solubilization and dispersing of the insoluble fat in the urine to homogenize and a reagent for a quantitative determination of the lipid component. That may also be a device for the measurement in a simple type where the reagent necessary for the measurement is contained in a carrier.

### (Means for Examination of Renal Failure)

Another means for solving the problems according to the present invention is a means for the examination of renal failure which is characterized in measuring a lipid component contained in urine. Up to now, examination of fat particles, etc. in urine for the detection of renal failure has been carried out by a troublesome means such as observation under a microscope. In the present invention, it has been found that the result of measurement of lipid contained in urine as well as lipoproteins in urine and/or apoproteins in urine obtained by the above-mentioned simple and convenient measuring method of the present invention for a lipid component in urine significantly suggests the relationship thereof with renal failure whereupon there has been achieved a simple and convenient examination method for renal failure using the measuring method of the present invention for a lipid component in urine. Further, the amount of the lipid component in urine is scored and degree of the renal failure of patients can be judged by such a score.

In the examination means of the present invention for renal failure, the above-mentioned measuring method for a lipid component in urine can be utilized. The lipid component contained in urine which is to be an object of the measurement is mostly contained in fat drop, fat body and/or fat particle in the urine. In the lipid component contained in fat drop, fat body and/or fat particle in the urine, the object for the measurement is at least one or more lipid component (s) selected from neutral fat and derivatives thereof, phospholipid and derivatives thereof, lipid peroxide, sterols, fatty acid, fatty acid salt, fatty acid ester, aliphatic alcohol, aliphatic aldehyde, glycolipid, sphingolipid, prostaglandin and carotenoid.

In an experimental example, cholesterol level and phospholipid level of the patient suffering from renal failure are measured. They are significantly in high levels as compared with the levels in normal persons and it has been found as a result thereof that quantitative determination of a lipid component in urine or at least cholesterol and/or phospholipid is useful for finding the renal failure.

Further, in the present invention, a combination of the measurement of lipid in urine with the measurement of lipoproteins in urine and/or apoproteins in urine is one of the appropriate embodiments and is able to give more appropriate judgment.

As such, the present invention provides a means for the examination of renal failure in which one or more lipid(s) in urine and lipid component(s) such as lipoproteins in urine and/or apoproteins in urine are measured and the result thereof is analyzed whereby detection of renal failure and judgment of its degree are now made possible.

In addition to the measurement for a lipid component in urine as such for the detection of renal failure and the judgment of degree of the failure, the present invention further discloses a combined utilization thereof with a method for the measurement or the detection of surface antigen of leukocyte contained in urine. The method for the measurement or the detection of surface antigen of leukocyte contained in urine may be introduced only for the sample where the lipid component in the urine shows a high value (upon scoring the measured result of lipid as well as lipoproteins and/or apoproteins). As a result of such a combination, more appropriate examination for renal failure is possible. Thus, when the measurement of a lipid component in urine is combined with the measurement or the detection of surface antigen of leukocytes contained in urine, then the detection of renal failure and the judgment of its degree can be carried out more appropriately.

In conducting the examination of renal failure by combining the measurement of a lipid component in urine with the measurement or the detection of surface antigen of leukocyte contained in urine, it is also possible that a part of urine is used for the above-mentioned measurement of lipid as well as of lipoproteins and/or apoproteins while another part thereof is subjected to a centrifugal separation to fractionate the urine sediment fraction and the surface antigens of leukocyte such as CD14, CD16 and 25F9 are measured and detected by known observation under a microscope, flow cytometry or the like. It is further possible that lipid as well as lipoproteins and/or apoproteins are measured using a part of urine sediment fraction while surface antigens of leukocyte are detected and measured by observation under a microscope, flow cytometry or the like using another part thereof. It is furthermore possible that a part of urine is centrifuged to fractionate a urine sediment and the surface antigens of leukocyte are measured and detected while another part of urine is subjected to a freeze melting and/or a treatment with surface-active agent either solely or jointly and then lipid as well as lipoproteins and/or apoproteins in such a step to destruct the cells in the urine are measured.

When the result obtained by the measurement of lipid in urine, the measurement of lipoproteins in urine and/or apoproteins in urine and the measurement or the detection of surface antigen of leukocyte contained in urine and the above included in the examination means for renal failure according to the present invention is combined and analyzed upon necessity, more appropriate judgment for renal failure is possible. The result of the measurement used for analysis may be appropriately selected. For example, the ratio of cholesterol to phospholipid may be used as an index or the said ratio may be combined with the result of measurement and detection of CD14. With regard to the result of measurement and detection of surface antigen of leukocyte contained in urine, plural results may be combined.

### Examples

The present invention will now be further illustrated by way of the following Examples although the present invention is not limited thereto.

### (Example 1)

Each of urines of normal person and patients suffering from renal failure, cholesterol concentration was measured. The urine was previously mixed with 10% Triton-100 in a ratio of 9:1 to prepare a urine sample.

In the measurement of cholesterol, two kinds of measuring reagents having the following compositions were prepared, 10 µL of the urine sample were taken, added to 250 µL of the reagent 1 and made to react at 37°C for 5 minutes, 100 µL of the reagent 2 were added and, after the addition, changes in the absorptions per 1 minute at the wavelength of 415 nm after 1 minute to 3 minutes were measured whereupon the cholesterol concentration was determined. The result is shown in Table 1.

| Reagent 1 | |
|---|---|
| PIPES | 20 mmol/L |
| KCl | 150 mmol/L |
| Triton X-100 | 0.3 w/v % |
| Sodium cholate | 0.2 w/v % |
| Thio NAD | 1.25 mmol/L |
| Bovine serum albumin | 0.1 w/v % |
| Cholesterol dehydrogenase | 3 U/ml |
| Cholesterol esterase | 5 U/ml |
| pH | 7.0 |

| Reagent 2 | |
|---|---|
| CHES | 175 mmol/L |
| Triton X-100 | 0.3 w/v % |
| Sodium cholate | 0.3 w/v % |
| β-NADH | 3.5 mmol/L |
| pH | 9.1 |

### (Example 2)

Concentration of phospholipid was measured for the urine sample prepared in Example 1. In the measurement of phospholipid, two kinds of measuring reagents having the following compositions were prepared, 10 µL of the urine sample were taken, added to 250 µL of the reagent 1 and made to react at 37°C for 5 minutes, 100 µL of the reagent 2 were added and, after the addition, changes in the absorptions per 1 minute at the wavelength of 546 nm after 1 minute to 3 minutes were measured whereupon the phospholipid concentration was determined. The result is shown in Table 1.

| Reagent 1 | |
|---|---|
| HEPES | 50 mmol/L |
| CaCl₂ | 1.0 mmol/L |
| Triton X-100 | 0.2 w/v % |
| Glycerol 3-dehydrogenase | 1.5 U/ml |
| Phospholipase A1 | 2.0 U/ml |
| Phospholipase A2 | 2.0 U/ml |
| Glycerophosphorylcholine phosphodiesterase | 0.6 U/ml |
| NADH | 0.5 mM |
| Phenol | 0.05% |
| Ascobic acid oxidase | 3 U/L |
| pH | 8.0 |

| Reagent 2 | |
|---|---|
| Phosphate buffer | 20 mmol/L |
| Glycero-3-phosphate oxidase | 200 U/ml |
| POD | 10 U/ml |
| 4-Aminoantipyrine | 2.0 mM |
| pH | 7.5 |

As a result, it was found that concentrations of cholesterol and phospholipid in urine of patients suffering from renal failure were clearly in high values as compared with normal persons and that renal failure was able to be examined by the measurement of at least cholesterol and/or phospholipid in urine. (Table 1) Cholesterol and Phospholipid in Urine of Normal Persons and Patients Suffering from Renal

| Failure | | |
|---|---|---|
| Urine Samples | Cholesterol (mg/dL) | Phospholipid (mg/dL) |
| Urine of Normal Person | 0.07 | 0.6 |
| Urine 1 of Patient Suffering from Renal Failure | 1.74 | 8.7 |
| Urine 2 of Patient Suffering from Renal Failure | 0.24 | 12.8 |
| Urine 2 of Patient Suffering from Renal Failure | 0.19 | 1.5 |
| Urine 3 of Patient Suffering from Renal Failure | 0.16 | 8.7 |

### (Example 3)

Concentrations of phospholipid, free cholesterol, cholesterol and apo A1 in urine samples of 122 cases (comprising 51 cases of IgA nephropathy [IgAN], 10 cases of diabetic nephropathy [DMN], 9 cases of nephrosclerosis [Nephrosclerosis], 6 cases of membranous nephropathy [MN], 7 case of membranous proliferative glomerulonephritis [MPGN], 12 cases of rapidly progressive nephropathy [RPGN], 3 cases of minimal change nephrotic syndrome [MCN], 12 cases of focal glomerular sclerosis [FGS], 5 cases of chronic renal failure [CRF], and 7 normal cases) were measured.

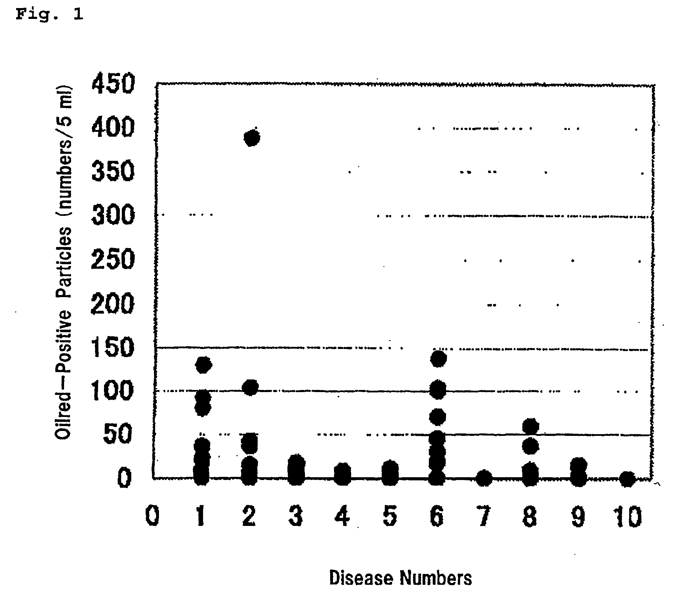

The result is shown in Figs. 1∼5. It was noted that any of those lipid-related markers showed a significantly high value as compared with normal urine in any of disease groups of the above renal failure and it is now apparent that, when lipid component in urine is measured, renal failure can be examined and diagnosed. In the meanwhile, numbers (in 5 mL) of Oilred 0-positive particles (fat particles in urine) as reported by Hotta, et al. were measured (Takashi Oda, Osamu Hotta, et al. , *Kidney International,* Vol. 53, 1190-1200, (1998)).

Fat particles showed especially high values in IgAN, RPGN and FGS but, on the contrary, in the case of MCN etc., they were as same as those in normal urine. As compared with the above, not only IgAN, RPGN and FGS but also other renal failures were found to be detected with a high sensitivity according to the method of the present invention and the usefulness of the present invention was confirmed.

Incidentally, measurement of cholesterol was carried out by the method mentioned in Example 1; measurement of phospholipid was carried out by the method mentioned in Example 2; and measurement of free cholesterol was carried out by the same operation as mentioned in Example 1 except that cholesterol esterase was removed from the reagent 1 shown in Example 1.

Measurement of apo A1 was carried out using ND "Kokusai" which was an apo A-I immune reagent.

Urine was previously mixed with 10% Triton X-100 in a ratio of 9:1 to prepare a urine sample. Operation was carried out in accordance with the Directions for Use except that the measurement was carried out using 10 µL of urine sample, 160 µL of the first reagent and 40 µL of the second reagent.

### (Example 4)

Urine samples (175 cases) collected from the persons receiving the periodical health examination were used for measuring the cholesterol in urine by a method mentioned in Example 1 to determine the referential value of cholesterol in urine of healthy persons. The cholesterol value in serum of healthy persons is usually 130∼250 mg/dL while the cholesterol value in urine was not more than 1/1000 of the cholesterol value in serum and was 0.01∼0.25 mg/dL.

As a control, measurement was conducted using a commercially available measuring kit for cholesterol in serum but cholesterol was unable to be measured in any of the samples. In view of the above, it is noted that, in the measurement of cholesterol in urine, a high-sensitivity measuring method using a method mentioned in Example 1, for example, of the present invention is necessary.

In the present invention, there is provided a method where a lipid component contained in urine can be measured in a simpler, more convenient and surer manner. By the utilization of that method, correlation of renal failure with the amount of lipid component contained in urine has been found and, with an object of detecting the renal failure, measurement of lipid in urine as well as lipoproteins in urine and/or apoproteins in urine has been found to be very effective. There are further provided a means for the examination of the degree of renal failure in a surer manner by analyzing the measured result of plural lipid components as well as lipoproteins and/or apoproteins in the measurement of lipid components in urine and also by combining with the measurement of surface antigen of leukocyte contained in urine.

## Claims

1. A means for the examination of renal failure which is **characterized in** measuring a lipid component contained in urine.

2. The means for the examination of renal failure according to claim 1, wherein the lipid component contained in the urine is measured by the use of enzyme acting the lipid component.

3. The means for the examination of renal failure according to claim 2, wherein the enzyme acting the lipid component is one or more enzyme(s) selected from enzymes which catalyze decomposition reaction, dehydration reaction or oxidation reaction.

4. The means for the examination of renal failure according to any of claims 1∼3, wherein the said lipid component is contained in at least one or more of fat drop, fat body and fat particle in urine.

5. The means for the examination of renal failure according to any of claims 1∼4, wherein the said lipid component is one or more lipid component(s) selected from neutral fat and derivatives thereof, phospholipid and derivatives thereof, lipid peroxide, sterols, fatty acid, fatty acid salt, fatty acid ester, aliphatic alcohol, aliphatic aldehyde, glycolipid, sphingolipid, prostaglandin and carotenoid.

6. A method for the measurement of a lipid component contained in urine, **characterized in that**, a means where insoluble fat in urine is solubilized or dispersed to homogenize is used.

7. The method for the measurement according to claim 6, wherein the means where insoluble fat in urine is solubilized or dispersed to homogenize is to use a surface-active agent.

8. The method for the measurement according to claim 6 or 7, wherein the lipid component is contained in at least one or more of fat drop, fat body or fat particle.

9. The method for the measurement according to any of claims 6∼8, wherein the said lipid component is one or more lipid component (s) selected from neutral fat and derivatives thereof, phospholipid and derivatives thereof, lipid peroxide, sterols, fatty acid, fatty acid salt, fatty acid ester, aliphatic alcohol, aliphatic aldehyde, glycolipid, sphingolipid, prostaglandin and carotenoid.

10. The method for the measurement according to any of claims 6∼9, wherein the said lipid component is any of the sterols and phospholipid.

11. The method for the measurement according to any of claims 6∼10, wherein the said surface-active agent has an action of solubilizing at least one or more of any of fat drop, fat body and fat particle and is able to solubilize, disperse, liberate and homogenize a lipid component contained in any of the said fat drop, fat body and. fat particle.

12. The method for the measurement according to any of claims 6∼11, wherein the said surface-active agent is nonionic surface-active agent, cationic surface-active agent, anionic surface-active agent, amphoteric surface-active agent or glycoside.

13. The method for the measurement according to any of claims 6∼12, wherein two or more of the said surface-active agents are jointly used.

14. The means for the examination of renal failure according to claim 1, wherein the method mentioned in any of claims 6∼13 is utilized.

15. The means for the examination of renal failure according to claim 1, wherein the method mentioned in any of claims 6∼13 is further utilized in addition to the means for the examination mentioned in claim 2 or 3.

16. The means for the examination of the renal failure according to any of claims 1∼3, 14 and 15, wherein the means is carried out in combination with at least one or more measurement(s) of lipoprotein in urine or apoprotein in urine.

17. Themeans for the examination of renal failure according to any of claims 1∼3 and 14∼16, wherein the means is carried out in combination with measurement or detection of surface antigen of leukocyte contained in urine.

18. A reagent for the measurement of lipid component in urine where the reagents necessary for the method for the measurement mentioned in any of claims 6∼13 are made in a kit or are composed of each single substance.
